# EUROPEAN PATENT APPLICATION

(11) **EP 0 655 449 A1**
(43) Date of publication of application: **31.05.1995**
(21) Application number: 94402127.8
(22) Date of filing: 23.09.1994
(51) Int. Cl.: C07D 401/14, A61K 31/505, C07D 211/40

(54) **A process for preparing N-( 2-pyrimidyl) piperazinyl butyl imides**

(30) Priority: 10.11.1993 KR 9323799
(71) Applicant: JIN RO LIMITED, Seoul 137-070 (KR)
(72) Inventor: Lee, Ki Hong, Songpa-ku, Seoul (KR); Kim, Dong Soo, Youngdeungpo-ku, Seoul (KR); Ryu, Dong Seung, Sosa-ku, Boucheon-si (KR); Cho, Young Woo, Dongdaemoon-ku, Seoul (KR); Ahn, Jin Hee, Joongrang-ku, Seoul (KR); Kim, Man Keun, Youngdeungpo-ku, Seoul (KR); Kim, Chul Hwan, Seocho-ku, Seoul (KR)
(74) Representative: Derambure, Christian

(57) **Abstract**

The present invention relates to a process for preparing N-(2-pyrimidyl)piperazinyl butyl imides with tranquilizing effect characterized by the following formula( I ) through a new intermediate. 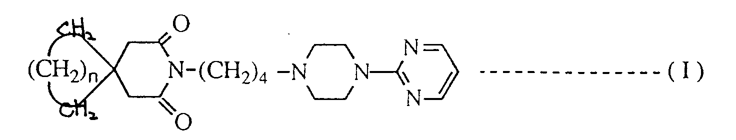
wherein n is equal to 0 or 2.

## Description

### Field of the Invention

The present invention relates to a process for preparing N-(2-pyrimidyl)piperazinyl butyl imides with tranquilizing effect characterized by the following formula(1) through a new intermediate. 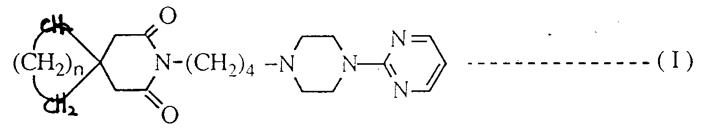
wherein n is equal to 0 or 2.

### Background of the Invention

Yao Hua Wu, et al, U.S. Patent No 3,717,634 disclose methods for synthesis of N-(hetero arcyclic)-piperazinylalkyl azaspiroalkanediones including the following.

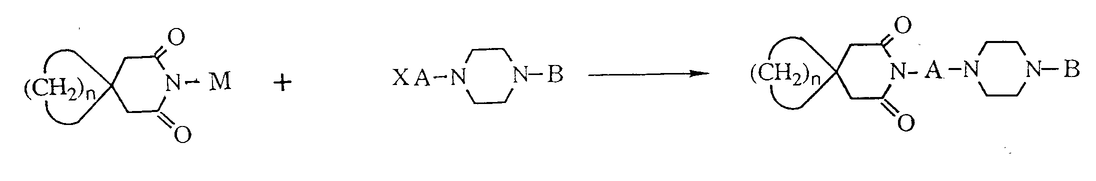

In the above reaction scheme , "n" is the integer 4 or 5, "M" comprises an alkali metal salt such as sodium or potassium : "X" inter alia is chlorine, bromine, iodine : and the symbol "A" connecting the spiroglutarimide and the N-(heteroarcyclic)piperazine represents a divalent alkylene chain of 2 to 6 carbon atoms inclusive. The symbol "B" represents inter alia various heterocyclic radicals including "2-pyrimidinyl". The instant process differs from the Wu, et al., U.S.Pat. No 3,717,634 Patent process above in that the N-(2-pyrimidyl)piperazinyl fragment is introduced by alkylation of performed N-(cis-4-halo or sulfonyloxy-2-butenyl)imide with N-(2-pyrimidyl)piperazine and hydrogenation is carried out.

### Summary of the Invention

It is an object of the present invention to provide a novel process of preparing N-(2-pyrimidyl)piperazinyl butyl imides.

The present invention involves the synthesis of novel N-(2-pyrimidyl)piperazinyl-2-butenyl imides which upon hydrogenation form N-(2-pyrimidyl)piperazinyl butyl imides (I) having psychopharmacologic properties.

Imides (I) can, if desired, be converted to suitable salt.

### Detailed Description of the Invention

The instant invention relates to a process for preparation of N-(2-pyrimidyl)piperazinyl butyl imide characterized by formula (I) 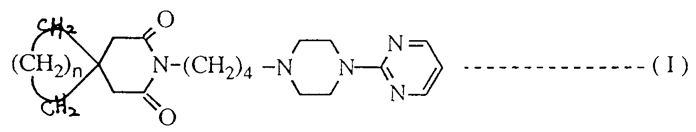
wherein n is equal to 0 or 2 obtained by hydrogenation of N-(2-pyrimidyl)piperazinyl-cis-2-butenylimide charaterized by formula (II) 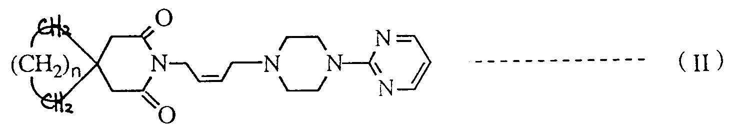
which comprises alkylating N-(cis-4-halo or sulfonyoxy-2-butenyl)imide of general formula (III) 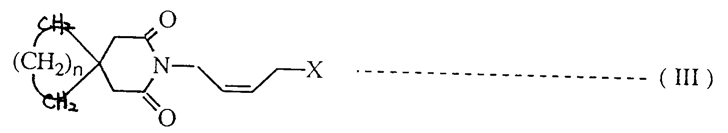
with a N-(2-pyrimidyl)piperazine characterized by formula (IV) 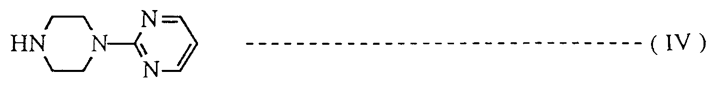
wherein X is chlorine, bromine, iodine, lower alkyl sulfonyloxy, phenyl or 4-methyl phenyl sulfonyloxy.

The compound of formula (III) comprises alkylating imide characterized by formula (V) 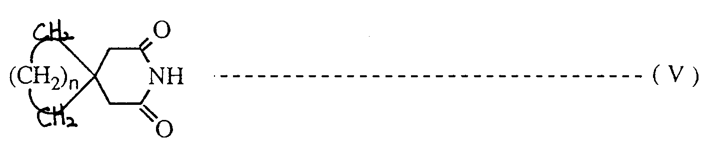
with cis-1,4-dihalo or disulfonyloxy-2-butene characterized by formula (VI) 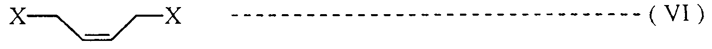
wherein n and X each are as defined previously.

The process of the invention is illustrated in the following reaction scheme.

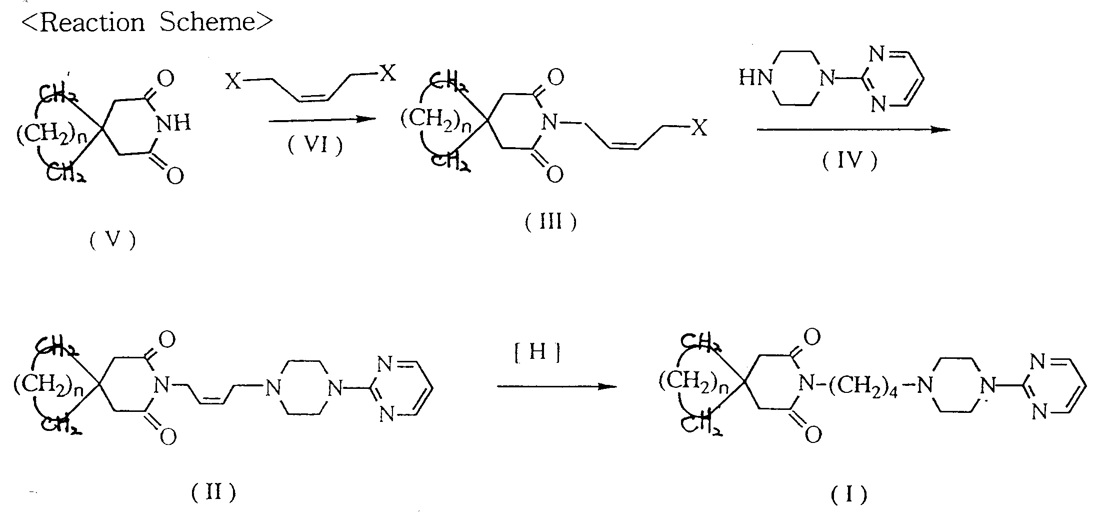

As shown reaction scheme, imide (V) is reacted with 1∼3 equivalent of cis-1,4-dihalo disulfonyloxy-2-butene (VI) in the presence of base to obtain mono-substituted compound (III).

The reactants are combined in a reaction inert liquid medium in the presence of inorganic base such as sodium bicarbonate, potassium bicarbonate, sodium carbonate or potassium carbonate particularly preferred.

Liquid reaction media temperature in the range of 40∼80°C are preferred. Suitable solvents include acetone as well as acetonitrile and dimethylformamide.

Imide (II) is prepared by condensation of mono-substituted compound of formula (III) with N-Pyrimidylpiperazine (IV) in the presence of inorganic base such as sodium bicarbonate and potassium bicarbonate, sodium carbonate or potassium carbonate.

The reaction can be carried out at 40∼80°C.

Suitable solvents are protic media such as t-butanol or s-butanol, aprotic media such as acetone, acetonitrile or dimethylformamide and aqueous solvent mixed with these organic solvent.

Title compound (I) is prepared by hydrogenation of N-(2-pyrimidyl)piperazinyl-cis-2-butenyl imide (II) over a metallic catalyst at 20∼50°C and 1∼30 psi in lower alkyl alcohol, ethyl acetate, tetrahydrofuran or acetone etc.

Metallic catalysts are Pd/c, Pd/BaCO₃, PtO₂, or Nickel boride particularly preferred.

The compound (I) of this invention forms physiologically acceptable salt with hydrochloride. The salt may be formed by conventional means, as by reacting the free base form (I) of the product with one equivalent of hydrochloride in a solvent such as ethanol or isopropyl alcohol. The process according to the present invention gives above 80% yield of N-(2-pyrimidyl)piperazinyl butyl imide (I) having psychopharmacologic properties under mild reaction condition.

The invention will be described in the following example, which should be understood to be illustrative and not limiting

### Example 1.

Preparation of 8-(cis-4-chloro-2-butenyl)-8-azaspiro[4,5]decan-7,9-dione (IV)

A mixture of 8-azaspiro[4,5]decan-7,9-dione (5.57g, 33.3mmole), cis-1,4-dichloro-2-butene(8.33g, 66.6mmole) and finely powdered potassium carbonate(11.5g, 83.3mmole) in 60ml of acetone is stirred and refluxed for 6 hours period. The reaction mixture is cooled to room temperature and filtered.

After concentration of the filtrate, xylene(15ml X 3) is added to the residue three times, condensed in vacuo. 70ml of ether is added to the residue and washed with 5% of NaHCO₃ aqueous solution and water, drying on MgSO₄ and filter. After concentration of the filtrates chromatography affords 8.09g of the desired product (95% of theoretical).
¹H NMR (CDCl₃) : δ 1.5(t, 4H), 1.7(m, 4H), 2.6(s, 4H), 4.3(d, 2H), 4.45(d, 2H), 5.6(m, 1H), 5.75(m, 1H)

### Example 2.

Preparation of 8-[4-{4-(2-pyrimidyl)-1-piperazinyl}-2-butenyl-8-azaspiro [4,5]decan-7,9-dione (VI)

A mixture of 8-(cis-4-chloro-2-butenyl)-8-azaspiro[4,5]decan-7,9-dione (8.52g, 33.3mmole), N-(2-pyrimidyl)piperazine (6.55g, 39.9mmole), potassium carbonate (9.2g, 66.6mmole) in 80ml of acetone is refluxed for 10 hour period and cooled to room temperature, filtered and concentrated.

To residue material 50ml of water and 7.32ml of c-H₂SO₄ are added. After washing with ether, aqueous solution is neutralized with 3.86g of sodium hydroxide and extracted with ethyl acetate (80ml X 3). After concentration of the solvent chromatography affords 12.14g of white solid.
M.P. : 78∼79°C
¹H NMR (CDCl₃) : δ 1.5(t, 4H), 1.7(m, 4H), 2.6(s, 8H), 3.3(d, 2H), 3.9(d, 4H), 4.4(m, 2H), 5.5(m, 1H), 5.7(m, 1H), 6.5(t, 1H), 8.3(d, 2H)

### Example 3.

Preparation of 8-[4-{4-(2-pyrimidyl)-1-piperazinyl}-2-butenyl]-8-azaspiro [4,5]decan-7,9-dione (VI)

A mixture of 8-azaspiro[4,5]decan-7,9-dione (50.16g, 0.3mole), cis-1,4-dichloro-2-butene (75g, 0.6mole) and finely powdered potassium carbonate (103.66g, 0.75mole) in 500ml of acetone is stirred and refluxed for 6 hour period, cooled, filtered and evaporated to dryness. Xylene (50ml X 3) is added to the residue three times,and condensed in vacuo.

Dissolving the residue in 600ml of acetone, N-(2-pyrimidyl)piperazine (59.13g, 0.36mole) and potassium carbonate (62.2g, 0.45mole) are added. The mixture is stirred for 6 hours at 55∼60°C and potassium carbonate (20.73g, 0.15mole) is added. Again the mixture is refluxed for 4 hours, cooled, filtered and then condensed in vacuo. To the residue 300ml of water, 55ml of c-HCl are added and stirred to clear solution.

The mixture is basified with aqueous sodium hydroxide, solids are collected and washed with water to provide 102.38g of 8[4{4-(2-pyrimidyl)-1-piperazinyl}-2-butenyl]-8-azaspiro[4,5]decan-7,9-di one (90% of theoretical).

### Example 4.

Preparation of 8-[4-{4-(2-pyrimidyl)-1-piperazinyl}-2-butenyl]-8-azaspiro[4,5]decan-7,9-dione hydrochloride

To a solution of 2g (0.5mmole) of 8-[4-{4-(2-pyrimidyl)-1-piperazinyl}-2-butenyl]-8-azaspiro[4,5]decan-7,9-dione in 20ml of tetrahydrofuran 0.3g of 5% Pd/BaCO₃ is added. The mixture is hydrogenated under 20 psi for 10 hour period, filtered and condensed to dryness. The solid taken up alcoholic cosolvent (IPA : EtOH = 5 : 1) and treated with ethanol solution of hydrochloride provides 2.2g of hydrochloride salt (98.2% of theoretical yield).
M.P. : 203∼206°C
¹H NMR (CDCl₃) : δ 1.5(t, 4H), 1.6∼1.8(m, 6H), 1.95(m, 2H), 2.6(s, 4H), 2.7(m, 2H), 3.1(m, 2H), 3.6(d, 2H), 3.8∼4.0(m, 4H), 4.9(d, 2H), 6.7(t, 1H), 8.4(d, 2H)

### Example 5.

Preparation of 8-[4-{4-(2-pyrimidyl)-1-piperazinyl}-2-butyl]-8-azaspiro [4,5] decan-7,9-dione hydrochloride

By substituting 5% Pd/c for Pd/BaCO₃ of example 4, the title compound can be prepared by the method described in that example.

### Example 6.

Preparation of 8-[4-{4-(2-pyrimidyl}-1-piperazinyl}-2-butyl]-8-azaspiro[4,5]decan-7,9-dione hydrochloride

By substituting PtO₂ for Pd/BaCO₃ of example 4, the title compound can be prepared by the method described in that example.

### Example 7.

Preparation of 8-[4-{4-(2-pyrimidyl)-1-piperazinyl}-2-butyl]-8-azaspiro[4,5]decan-7,9-dione hydrochloride

To a solution of 8-[4-{4-(2-pyrimidyl)-1-piperazinyl}-2-butenyl]-8-azaspiro[4,5]decan-7,9-dione in 15ml of methanol 10mg of fresh nickel boride is added. The mixture is hydrogenated under 20 psi for 7 hour period and filtered and condensed to dryness. Water is added to the residue and the mixture acidified with d-HCl solution, washed with ether. The mixture is basified with aqueous sodium hydroxide. Precipitates are collected and washed with water to provide 2.0g of 8-[4-{4-(2-pyrimidyl)-1-piperazinyl}-2-butyl]-8-azaspiro[4,5]decan-7,9-dione as the free base.

The free base taken up in isopropyl alcohol - ethanol ( 5 : 1 ) solution and treated with ethanolic hydrochloride solution provides the hydrochloride salt (2.06g, 93% of theoretical yield).

## Claims

1. A process for preparing N-(2-pyrimidyl)piperazinyl butyl imide of formula (I) characterized in preparing N-(2-pyrimidyl) piperazinyl-cis-2-butenyl imide of formula(III) by alkylating imide of formula(V) with cis-1,4-dihalo or disulfonyloxy-2-butene of formula(VI), preparing N-(2-pyrimidyl) piperazinyl-cis-2-bytenyl imide of formula(II) by alkylating said formula(III) with N-(2-pyrimidyl) piperazine of formula (IV), and hydrogenating said formula(II). 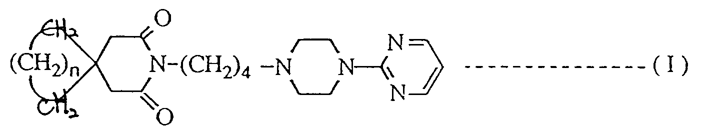 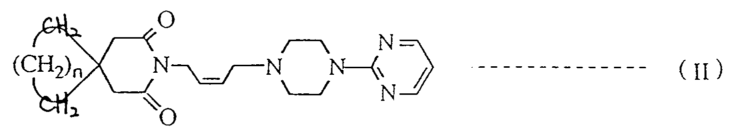 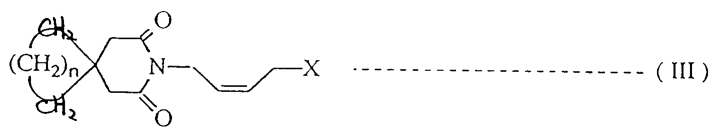 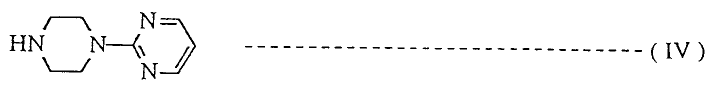 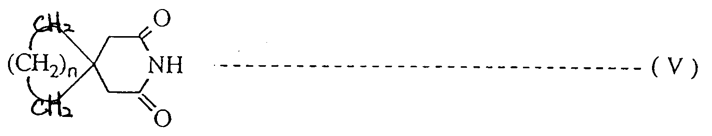 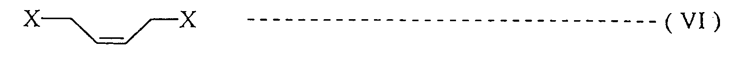 wherein n is equal to 0 or 2 and X is chlorine, bromine, iodine, lower alkylsulfonyloxy, phenyl or 4-methylphenyl sulfonyloxy.

2. The process for preparing formula (I) as defined in claim 1, wherein reaction for preparing said formula (II) and said formula (III) is maintained at 40∼80°C in the presence of inorganic base sodium bicarbonate, potassium bicarbonate, sodium carbonate or potassium carbonate.

3. The process for preparing formula (I) as defined in claim 1, wherein the hydrogenation of said formula(II) is carried out under pressed hydrogen gas between 1 psi and 30 psi.

4. The process for preparing formula (I) as defined in claim 1, wherein metallic catalyst is Pd/c, Pd/BaCO₃, PtO₂ or Nickel boride.

5. A compound characterized by formula (III) 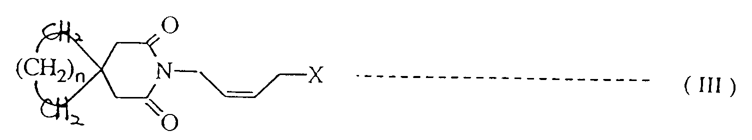 which is useful intermediate for preparation of N-(2-pyrimidyl)piperazinyl butyl imide of formula (I) 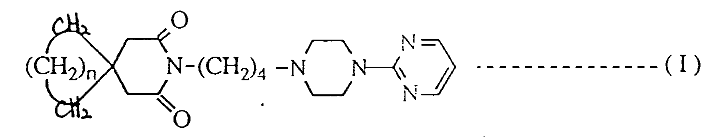 wherein n and X are as defined in claim 1.
